# EUROPEAN PATENT APPLICATION

(11) **EP 2 206 700 A1**
(43) Date of publication of application: **14.07.2010**
(21) Application number: 08254163.2
(22) Date of filing: 24.12.2008
(51) Int. Cl.: C07D 213/75, A61K 31/44

(54) **Crystalline form of flupirtine ((2-amino-6-(4-fluoro-benzylamino)-pyridin-3-yl)-carbamic acid ethyl ester)**

(71) Applicant: AWD.pharma GmbH & Co.KG, 01445 Radebeul (DE)
(72) Inventor: Hoock, Christoph, 01157 Dresden (DE); Mundorfer, Tina, 10000 Zagreb (HR); Leksic, Edislav, 10000 Zagreb (HR); Kwokal, Ana, 10000 Zagreb (HR); Zegarac, Miroslav, 10020 Zagreb (HR)
(74) Representative: Nachshen, Neil Jacob

(57) **Abstract**

The present invention is concerned with a new polymorphic form of [2-amino-6-(4-fluoro-benzylamino)-pyridin-3-yl]-carbamic acid ethyl ester, 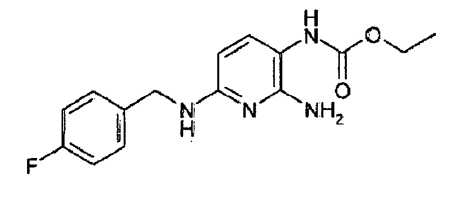 processes for preparing the new polymorphic forms, pharmaceutical compositions containing them, therapeutic uses thereof and methods of treatment employing them.

## Description

The present invention is concerned with a new polymorphic form of [2-amino-6-(4-fluoro-benzylamino)-pyridin-3-yl]-carbamic acid ethyl ester, processes for preparing the new polymorphic forms, pharmaceutical compositions containing them, therapeutic uses thereof and methods of treatment employing them.

[2-Amino-6-(4-fluoro-benzylamino)-pyridin-3-yl]-carbamic acid ethyl ester also known as flupirtine can be represented by the following structural formula:

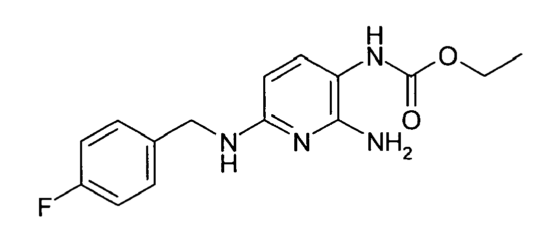

Flupirtine is a centrally acting non-opioid analgesic which is devoid of the typical side effects of natural or synthetic opioids, such as respiratory depression, constipation, tolerance, physical and/or psychological dependence and liability to cause addiction. It is also a muscle-relaxant. Although flupirtine does not appear to bind to any of the as yet identified NMDA receptor complex associated binding sites, it has several functional NMDA antagonistic properties. Flupirtine has also been shown to increase the expression of the protein Bcl-2, which is known to inhibit apoptosis (programmed cell death).

As a result of these many and varied activities, flupirtine has a unique spectrum of pharmacological activity. Flupirtine has utility in the treatment and prevention of acute and chronic pain including neuropathic pain, nerve pain, cancer pain, vasomotor and migraine headaches, post-operative pain, post-traumatic pain, burn pain, erosion pain, dysmenorrhoea, dental pain and the pain associated with degenerative and inflammatory joint disease.

Flupirtine also has utility in the treatment and prevention of muscular tension, muscle spasm and muscle stiffness. It is particularly useful in the treatment of back pain.

Additionally, flupirtine also exerts potent cyto- and neuroprotective effects and has utility in the treatment and prevention of neurodegenerative disorders such as Parkinson's disease, dementia including Alzheimer's disease, Huntington's chorea, multiple sclerosis, amyotrophic lateral sclerosis, encephalopathy including AIDS related encephalopathy, Creutzfeldt-Jakob disease including classical and new-variant types and Batten disease.

Flupirtine also has utility in the treatment and prevention of diseases of the eye such as maculopathy including senile macular degeneration, diabetic retinopathy, glaucoma and retinitis pigmentosa.

EP 199 951 describes a process for preparing [2-Amino-6-(4-fluoro-benzylamino)-pyridin-3-yl]-carbamic acid ethyl ester.

Polymorphic forms of a drug substance can have different chemical and physical properties, including melting point, chemical reactivity, apparent solubility, dissolution rate, optical and mechanical properties, vapour pressure, and density. These properties can have a direct effect on the ability to process and/or manufacture a drug substance and a drug product, as well as on drug product stability, dissolution, and bioavailability. Thus, polymorphism can affect the quality, safety, and efficacy of a drug product.

There are a number of methods that can be used to characterise polymorphs of a drug substance. Demonstration of a non-equivalent structure by single crystal X-ray diffraction is currently regarded as the definitive evidence of polymorphism. X-ray powder diffraction can also be used to support the existence of polymorphs. Other methods, including microscopy, thermal analysis (e.g., differential scanning calorimetry, thermal gravimetric analysis, and hot-stage microscopy), and spectroscopy (e.g., infrared (IR) and near infrared (NIR), Raman and solid-state nuclear magnetic resonance [ssNMR]) are also helpful to further characterise polymorphic forms.

Drug substance polymorphic forms can exhibit different chemical, physical and mechanical properties as referred to above, including aqueous solubility and dissolution rate, hygroscopicity, particle shape, density, flowability, and compactibility, which in turn may affect processing of the drug substance and/or manufacturing of the drug product. Polymorphs can also exhibit different stabilities. The most stable polymorphic form of a drug substance is often chosen during drug development based on the minimal potential for conversion to another polymorphic form and on its greater chemical stability. However, a meta-stable form can alternatively be chosen for various reasons, including better bioavailability. One of the most important physical properties of pharmaceutical polymorphs is their solubility in aqueous solution, particularly their solubility in the gastric juices of a patient. For example, where absorption through the gastrointestinal tract is slow, it is often desirable for a drug that is unstable to conditions in the patient's stomach or intestine to dissolve slowly so that it does not accumulate in a deleterious environment. On the other hand, where the effectiveness of a drug correlates with peak bloodstream levels of the drug, and provided the drug is rapidly absorbed by the GI system, then a more rapidly dissolving form is likely to exhibit increased effectiveness over a comparable amount of a more slowly dissolving form.

Typically pharmaceutical forms of active substances are delivered, where the molecule is capable of being so formed, in the form of a salt. Salt forms are typically preferred since they provide for easily to handle solids that are more likely to be crystalline and also consequently are more likely to be physically stable. In addition salt forms can be created, by the careful choice of the counter ion, that are more or are less soluble that the free base or acid, and have quicker or slower dissolution profiles.

There is now provided by the present invention, therefore, polymorphic forms of the free base of flupirtine with advantageous properties.

The prior art forms of flupirtine concentrate on salt forms. In particular the form that is commercially available as Katadalone™ is the maleate salt of flupirtine. We have now surprisingly found that the free base of flupirtine is capable of forming different polymorphic forms and that certain polymorphic forms of the free base flupirtine exhibit beneficial properties that enable the free base of flupirtine to be utilised in pharmaceutical compositions and, in particular, provide advantages over available flupirtine forms.

More particularly, there is provided by the present invention polymorphic forms III of flupirtine.

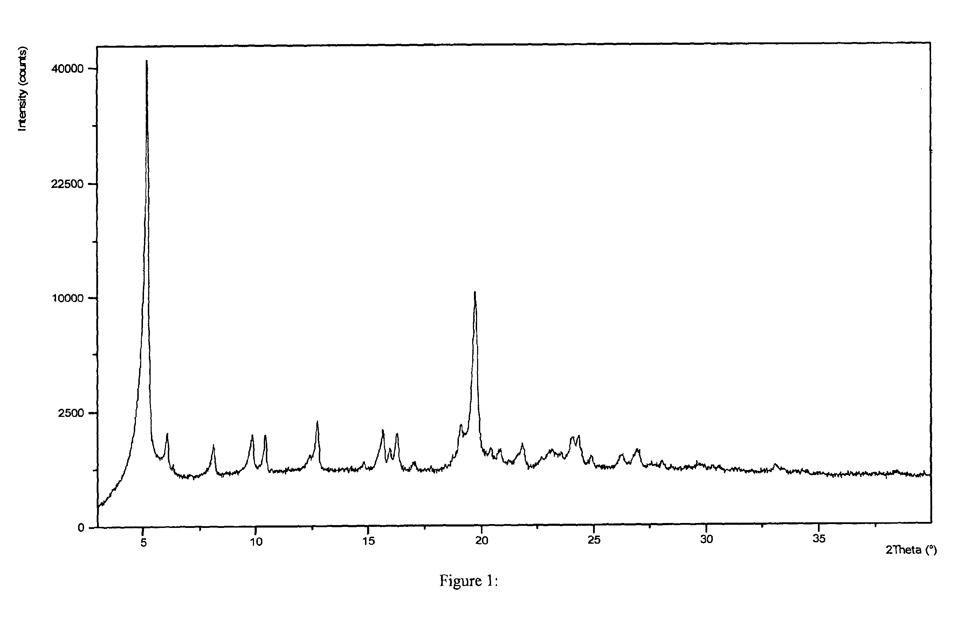

The crystalline structure of polymorph III of flupirtine according to the present invention is characterised as having an X-ray powder diffraction pattern, or substantially the same X-ray powder diffraction pattern, as is shown in Figure 1.

Polymorph III of flupirtine according to the present invention is further characterised as having characteristic peaks (2θ°) selected from one or more of the following: 5.2±0.2°, 12.8±0.2°, 19.8±0.2°, and 21.8±0.2°. Further peaks (2θ) associated with polymorph III of flupirtine according to the present invention are selected from one or more of the following: 6.1±0.2°, 8.1±0.2°, 9.9±0.2°, 10.4±0.2°, 15.7±0.2°,16.3° ±0.2°, and 24.3±0.2°

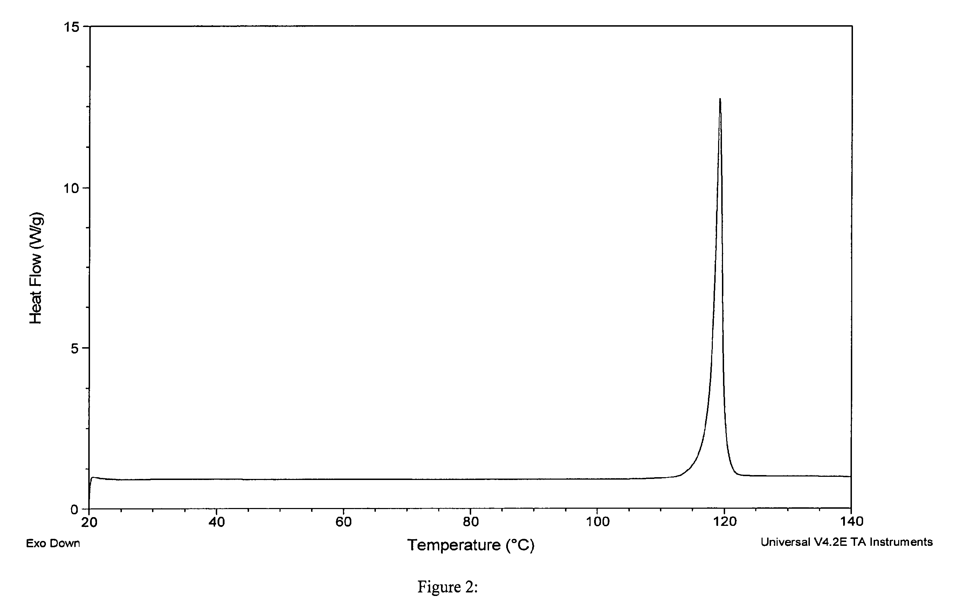

Polymorph III of flupirtine according to the present invention is further characterised by a typical differential scanning calorimetry (DSC) thermogram as is shown in Figure 2. Polymorph III of flupirtine has a characteristic DSC melting endotherm at about 119±2 °C.

Polymorph III is obtainable by dissolving, fully or partly, flupirtine with high boiling solvents like *p*-xylene methyl ethyl ketone, *i*-butanol, acetonitrile, 1-octanol, 1-pentanol, ethylene glycol etc.

There is also provided by the present invention processes for preparing polymorphic forms of flupirtine substantially as hereinbefore described.

In certain embodiments of a process as provided by the present invention a polymorphic form of flupirtine substantially as hereinbefore described is formed by solvent crystallisation.

As a result of these many and varied activities, the polymorphic forms of flupirtine, as provided by the present invention, have a unique spectrum of pharmacological activity. They have utility in the treatment and prevention of acute and chronic pain including neuropathic pain, nerve pain, cancer pain, vasomotor and migraine headaches, post-operative pain, post-traumatic pain, burn pain, erosion pain, dysmenorrhoea, dental pain and the pain associated with degenerative and inflammatory joint disease.

They also have utility in the treatment and prevention of muscular tension, muscle spasm and muscle stiffness. They are particularly useful in the treatment of back pain.

Additionally, the polymorphic forms of flupirtine, as provided by the present invention, exert potent cyto- and neuroprotective effects and have utility in the treatment and prevention of neurodegenerative disorders such as Parkinson's disease, dementia including Alzheimer's disease, Huntington's chorea, multiple sclerosis, amyotrophic lateral sclerosis, encephalopathy including AIDS related encephalopathy, Creutzfeldt-Jakob disease including classical and new-variant types and Batten disease.

They also have utility in the treatment and prevention of diseases of the eye such as maculopathy including senile macular degeneration, diabetic retinopathy, glaucoma and retinitis pigmentosa.

They also have utility in the treatment and prevention of myocardial ischaemia and infarction, cerebral ischaemia and infarction, shock, tinnitus and hepatitis.

The present invention further provides, therefore, pharmaceutical compositions comprising a therapeutically effective dose of a polymorphic form of flupirtine according to the invention, together with a pharmaceutically acceptable carrier, diluent or excipient therefor. Excipients are chosen according to the pharmaceutical form and the desired mode of administration.

As used herein, the term "therapeutically effective amount" means an amount of a polymorphic form of flupirtine according to the invention, which is capable of preventing, ameliorating or eliminating a disease state for which administration of a centrally acting non-opioid analgesics, a muscle-relaxant, a functional NMDA antagonist or a substance that increases the expression of the protein Bcl-2 is indicated.

By "pharmaceutically acceptable" it is meant that the carrier, diluent or excipient is compatible with a polymorphic form of flupirtine according to the invention, and not deleterious to a recipient thereof.

In the pharmaceutical compositions of the present invention for oral, sublingual, subcutaneous, intramuscular, intravenous, topical, intranasal, transdermal, ophthalmic or rectal administration, a polymorphic form of flupirtine according to the present invention is administered to animals and humans in unit forms of administration, mixed with conventional pharmaceutical carriers, for the prophylaxis or treatment of the above disorders or diseases. The appropriate unit forms of administration include forms for oral administration, such as tablets, gelatin capsules, soft capsules, powders, granules and solutions or suspensions to be taken orally, forms for sublingual, buccal, or intranasal administration, forms for subcutaneous, intramuscular or intravenous administration and forms for rectal administration. For topical application, a polymorphic form of flupirtine according to the present invention can be used in creams, ointments or lotions.
Although the most suitable route in any given case will depend on the nature and severity of the condition being treated, the most preferred routes of the present invention are rectal and oral.

To achieve the desired prophylactic or therapeutic effect, the dose of a polymorphic form of flupirtine according to the present invention can vary between 0.1 and 50 mg per kg of body weight per day. Each unit dose can contain from 10 to 1000 mg, preferably 30 to 700 mg, of a polymorphic form of flupirtine according to the present invention in combination with a pharmaceutical carrier. This unit dose can be administered 1 to 5 times a day so as to administer a daily dosage of 10 to 1000 mg, preferably 30 to 700 mg.

The Dosages may be conveniently presented in unit dosage form and prepared by any of the methods well known in the art of pharmacy. Dosage forms include solid dosage forms, like tablets, powders, capsules, suppositories, sachets, troches and lozenges as well as liquid suspensions and elixirs. While the description is not intended to be limiting, the invention is also not intended to pertain to true solutions of flupirtine polymorphs III whereupon the properties that distinguish the solid forms of flupirtine salts are lost. However, the use of the novel forms to prepare such solutions (e.g. so as to deliver, in addition to flupirtine, a solvate to said solution in a certain ratio with a solvate) is considered to be within the contemplated invention.

When a solid composition in the form of tablets is prepared, a polymorphic form of flupirtine according to the present invention is mixed with a pharmaceutical vehicle such as gelatin, starch, lactose, magnesium stearate, talc, gum arabic or the like. The tablets can be coated with sucrose, a cellulose derivative or other appropriate substances, or else they can be treated so as to have a prolonged or delayed activity and so as to release a predetermined amount of active principle continuously.

A preparation in the form of gelatin capsules can be obtained by mixing a polymorphic form of flupirtine according to the present invention with a diluent and pouring the resulting mixture into soft or hard gelatin capsules. Other conventional encapsulating material is equally suitable. The addition of an antioxidant can be necessary if the encapsulating material or the secondary package material (like blisters of containers) are permeable for oxygen.

Sucrose esters are suitable for preparing controlled release formulations of flupirtine.
A preparation in the form of a syrup or elixir or for administration in the form of drops can contain a polymorphic form of flupirtine according to the present invention typically in conjunction with a sweetener, optionally antiseptics such as methylparaben and propylparaben, as well as a flavoring and an appropriate color.

Water-dispersible granules or powders can contain a polymorphic form of flupirtine according to the present invention mixed with dispersants or wetting agents, or suspending agents such as polyvinylpyrrolidone, as well as with sweeteners, antioxidants or taste correctors.

Rectal administration is effected using suppositories prepared with binders which melt at the rectal temperature, for example polyethylene glycols.

Parenteral administration is effected using aqueous suspensions, isotonic saline solutions or sterile and injectable solutions which contain pharmacologically compatible dispersants and/or wetting agents, for example propylene glycol or butylene glycol.

A polymorphic form of flupirtine according to the present invention can also be formulated as microcapsules, with one or more carriers or additives if appropriate.

Suitable antioxidants for flupirtine formulations are non toxic excipients such as vitamins A, C and E and their derivatives or gallates, buthylhydroxyanisole, butylhydroxytoluol, sulfites, lecithine, EDTA and their derivatives.

There is also provided by the present invention a polymorphic form of flupirtine substantially as herein before described for use in therapy.

The present invention further provides a polymorphic form of flupirtine substantially as hereinbefore described, for the treatment of a disease state prevented, ameliorated or eliminated by the administration of a centrally acting non-opioid analgesic, a muscle-relaxant, a functional NMDA antagonist and/or an apoptosis inhibitor. More specifically, the present invention provides a polymorphic form of flupirtine, substantially as hereinbefore described, for use in the manufacture of a medicament for treating and preventing a number of disorders including acute and chronic pain, including neuropathic pain, nerve pain, cancer pain, vasomotor and migraine headaches, post-operative pain, post-traumatic pain, burn pain, erosion pain, dysmenorrhoea, dental pain, the pain associated with degenerative and inflammatory joint disease, muscular tension, muscle spasm, muscle stiffness, back pain, tension headache, neurodegenerative disorders such as Parkinson's disease, dementia including Alzheimer's disease, Huntington's chorea, multiple sclerosis, amyotrophic lateral sclerosis, encephalopathy including AIDS related encephalopathy, Creutzfeldt-Jakob disease including classical and new-variant, Batten disease, diseases of the eye such as maculopathy including senile macular degeneration, diabetic retinopathy, glaucoma, retinitis pigmentosa, myocardial ischaemia and infarction, cerebral ischaemia and infarction, shock, tinnitus and hepatitis.

The present invention also provides a method of treating a disease state prevented, ameliorated or eliminated by the administration of a centrally acting non-opioid analgesic, a muscle-relaxant, a functional NMDA antagonist and/or an apoptosis inhibitor in a patient in need of such treatment, which method comprises administering to the patient a therapeutically effective amount of a polymorphic form of flupirtine, substantially as hereinbefore described.

The present invention also provides a corresponding method of treatment, which comprises administering to a patient a therapeutically effective amount of a polymorphic form of flupirtine, substantially as hereinbefore described, so that the administered polymorphic form of flupirtine according to the present invention, provides an enhanced therapeutic effect to the patient, compared to the therapeutic effect provided by corresponding administration of the existing polymorphic forms of flupirtine.

The present invention can be further illustrated by the following Figures and non-limiting Examples.

With reference to the Figures, these are as follows:
Figure 1: X-ray powder diffraction pattern of flupirtine polymorph III according to the present invention obtained by using a Philips X'Pert PRO with Cu*K*α radiation in 2θ = 3-40 ° range.
Figure 2: Differential Scanning Calorimetry (DSC) thermogram of flupirtine polymorph III obtained by using a DSC Pyris 1 manufactured by Perkin- Elmer. The experiment was done under a flow of nitrogen (35 ml/min) and heating rate was 10 °C/min. A standard sample pan was used.

The following examples are for the purpose of illustration of the invention only and are not intended in any way to limit the scope of the present invention. It will thus be readily apparent to one skilled in the art that varying substitutions and modifications may be made to the invention disclosed herein without departing from the scope and spirit of the invention. Thus, it should be understood that although the present invention has been specifically disclosed by preferred embodiments and optional features, modification and variation of the concepts herein disclosed may be resorted to by those skilled in the art, and that such modifications and variations are considered to be falling within the scope of the invention.

The X-ray powder diffraction analysis was performed by the experimental methods detailed in Table 1, where XRPD analysis was carried out on Philips X'Pert PRO diffractometer using CuKα1 radiation and the pharmaceutical formulation premix was subjected to thermal analysis by the experimental procedure and condition listed in Table2.

**Table 1: X-ray powder diffraction (XRPD) experimental conditions**

| Sample holder preparation | Samples after being powdered in a mortar and pestle are applied directly on silicon PW1817/32 "zero background" holder |
|---|---|
| Instrument | Philips X'Pert PRO |
| Goniometer | PW3050/60 |
| Generator | PW3040; 45 kV, 40 mA |
| X-Ray tube | PW3373/00; Cu anode LFF |
| Focus | Linear |
| Sample stage | PW3072/60 or PW3064 |
| Scan angle range (2θ) | 3 - 40° |
| Scan mode | Continuous absolute scan |
| Step size (2θ) | 0.016° |
| Time per step | 100 seconds |
| X-ray radiation | λ(Cu*K*α1) = 1.540598 Å |
| Primary soller slit | 0.04 rad |
| PDS | Fixed, divergence 0.5° |
| Primary mask | 10 mm |
| Secondary soller slit | 0.04 rad |
| Monochromator | Inc. Beam α1 Cu/Co for reflection mode |
| Detector | X'Celerator (2.022° 2θ) |
| Control program | X'Pert Data Collector |
| Temperature | 293±2 K |

**Table 2. Experimental conditions for DSC analysis.**

| | |
|---|---|
| 1 | Equipment Q 1000 MDSC TA instruments |
| 2 | Dynamic flow of nitrogen 50 ml/min |
| 3 | Heating rate of 10 °C/min |
| 4 | Standard closed aluminum pan |
| 5 | Sample mass about 1 mg |
| 6 | Temperature range from -20/ or 20 °C up to 200 °C |

### EXAMPLE 1

### Preparation of flupirtine form III

30 mg of flupirtine base was dissolved in 0,5 mL of p-xylene while heating. Resulting solution was placed to crystallize at room temperature in closed bottle. The product was filtered and dried overnight at room temperature. 28 mg of yellowish product was obtained.

## Claims

1. Polymorph III of flupirtine **characterised** as having an X-ray powder diffraction pattern, or substantially the same X-ray powder diffraction pattern, as shown in Figure 1.

2. Polymorph III of flupirtine **characterised** as having characteristic peaks (2θ): 5.2 +0.2°, 12.8 +0.2°, 19.8+0.2°, and 21.8±0.2°.

3. Polymorph III of flupirtine **characterised by** a differential scanning calorimetry (DSC) thermogram as shown in Figure 2.

4. A polymorph according to claim 3 having a characteristic differential scanning calorimetry (DSC) a melting endotherm of 119±2 °C.

5. A pharmaceutical composition comprising a therapeutically effective dose of a polymorphic form of flupirtine according to any of claims 1 to 4 together with a pharmaceutically acceptable carrier, diluent or excipient therefor.

6. A pharmaceutical composition according claim 5 suitable for use as an antiphlogestic agent or an analgesic agent.

7. A process for the production of polymorph III of [2-Amino-6-(4-fluoro-benzylamino)-pyridin-3-yl]-carbamic acid ethyl ester comprising the steps:
(i) treating fl upirtine in a solvent or solvent mixture which supports formation of flupirtine polymorph III,
(ii) obtaining flupirtine polymorph III from the solution of step (i).

8. Process according claim 7 wherein the solvent is selected from *p*-xylene methyl ethyl ketone, *i-*butanol, acetonitrile, 1-octanol, 1-pentanol, ethylene glycol.

9. Use of a polymorphic form of flupirtine according to any of claims 1 to 4, in the preparation of a medicament for the treatment of a disease state prevented, ameliorated or eliminated by the administration of a centrally acting non-opioid analgesics, a muscle-relaxant, a functional NMDA antagonist or a substance that increases the expression of the protein Bcl-2.

10. Use according to claim 9, wherein the disease state is selected from the group consisting of acute and chronic pain, including neuropathic pain, nerve pain, cancer pain, vasomotor and migraine headaches, post-operative pain, post-traumatic pain, burn pain, erosion pain, dysmenorrhoea, dental pain, the pain associated with degenerative and inflammatory joint disease, muscular tension, muscle spasm, muscle stiffness, back pain, neurodegenerative disorders such as Parkinson's disease, dementia including Alzheimer's disease, Huntington's chorea, multiple sclerosis, amyotrophic lateral sclerosis, encephalopathy including AIDS related encephalopathy, Creutzfeldt-Jakob disease including classical and new-variant, Batten disease, diseases of the eye such as maculopathy including senile macular degeneration, diabetic retinopathy, glaucoma, retinitis pigmentosa, myocardial ischaemia and infarction, cerebral ischaemia and infarction, tinnitus.

11. Use according to claim 10, wherein the disease state is back pain or tension headache.
